# EUROPEAN PATENT APPLICATION

(11) **EP 0 956 898 A2**
(43) Date of publication of application: **17.11.1999**
(21) Application number: 99108295.9
(22) Date of filing: 28.04.1999
(51) Int. Cl.: B01J 23/34, B01J 21/08, C07C 235/06, C07C 231/06

(54) **Mn and Si-containing catalyst for hydrating cyanohydrin**

(30) Priority: 13.05.1998 JP 13054398
(71) Applicant: MITSUBISHI GAS CHEMICAL COMPANY, INC., Tokyo 100-0005 (JP)
(72) Inventor: Sugano, Yuichi Mitsubishi Gas Chemical Cpy, Inc., Niigata-shi, Niigata-ken (JP); Uchiyama, Takako Mitsubishi Gas Chemical Cpy, Inc., Niigata-shi, Niigata-ken (JP); Abe, Takafumi Mitsubishi Gas Chemical Cpy, Inc., Niigata-shi, Niigata-ken (JP)
(74) Representative: Türk, Gille, Hrabal

(57) **Abstract**

A catalyst for hydrating cyanohydrin, comprising a manganese oxide as a main component and a silicon compound. The mechanical strength of the manganese oxide conventionally known as a catalyst for hydrating a cyanohydrin is improved by adding the silicon compound. The mechanical strength can be improved without reducing the catalytic activity, when the silicon compound is added at any stage before separating the manganese oxide from a suspension containing it.

## Description

### BACKGROUND OF THE INVENTION

The present invention relates to a catalyst for use in hydrating cyanohydrins and a production method of a hydroxycarboxylic acid amide using the catalyst.

The hydroxycarboxylic acid amide is an important starting material for producing a hydroxycarboxylic ester and an unsaturated carboxylic ester, and is produced by hydrating a cyanohydrin such as acetone cyanohydrin. Therefore, it is industrially significant to develop a hydration catalyst for use in the production of the hydroxycarboxylic acid amide.

A highly active and selective long-life hydration catalyst mainly composed of a manganese oxide and the preparation method thereof are disclosed in Japanese Patent Application Laid-Open Nos. 63-57534, 63-57535, 3-68447, 3-93761, 5-170720, 6-269666, 9-19637, and 9-24275. The production of the hydroxycarboxylic acid amide by hydrating a cyanohydrin has been usually performed by a batch or flow method using a fixed bed catalyst formed by shaping the proposed catalyst or a suspension bed catalyst in the form of powder, granule or fine sphere.

The catalyst to be used in such a manner is required to have a sufficient mechanical strength in addition to the high activity and high selectivity. However, the known catalyst mainly composed of a manganese oxide suffers from fracture, powdering, abrasion, etc. when it is used as a fixed bed catalyst for hydrating a cyanohydrin in a flow reactor. As a result thereof, the reactant liquid flows unevenly to fail in obtaining expected results. In a severe case, the flow of the reaction liquid is stopped to result in a forced discontinuance of the reaction operation. When the known catalyst mainly composed of a manganese oxide is used as a slurry catalyst for hydrating a cyanohydrin in a batch or flow reactor, fracture, powdering, abrasion, etc. also occur to make it difficult to recover and reuse the catalyst.

### SUMMARY OF THE INVENTION

Accordingly, an object of the present invention is to provide an industrially useful catalyst for hydrating a cyanohydrin, being free from the above drawbacks in the prior art. Another object of the present invention is to provide a method of producing a hydroxycarboxylic acid amide using the catalyst.

### DETAILED DESCRIPTION OF THE INVENTION

The inventors have found that the mechanical strength of the catalyst mainly composed of a manganese oxide has been extremely enhanced by the addition of a silicon compound, although the catalytic activity tends to be slightly lowered. As a result of further study, the inventors have found that the catalytic activity is not lowered even by the addition of the silicon compound when it is added in the production of the catalyst at any stage prior to a separation step for obtaining a wet cake of a manganese oxide. The present invention has accomplished based on these findings.

Thus, the present invention provides a catalyst for hydrating a cyanohydrin, comprising a manganese oxide as a main component and a silicon compound. The present invention further provides a method of producing a hydroxycarboxylic acid amide, which comprises a step of hydrating a cyanohydrin over the catalyst.

### PREFERRED EMBODIMENTS OF THE INVENTION

The method of the present invention will be described below in detail. The manganese oxide constituting the substantial part of the catalyst of the present invention is preferably manganese dioxide. In the present invention, manganese oxides represented by the formulae from MnO_{1.7} to MnO₂ and having various crystalline structures such as α, β, γ, δ, ε, etc. are collectively referred to as "manganese dioxide." The manganese dioxide has a quite complicated and varying structure due to the transition between the phases and change in the crystallinity. The manganese oxide usable in the present invention may include the manganese dioxide having such a complicated and varying structure. Although a manganese dioxide naturally occurring may be used, a manganese oxide prepared by a method of oxidizing manganese (II), a method of reducing manganese (VII), or a combined method thereof is preferably used for preparing the catalyst of the present invention.

Known method of reducing manganese (VII) may include a method of reducing a permanganate by a hydrohalogenic acid (Japanese Patent Application Laid-Open No. 63-57535), a method of reducing a permanganate by a polybasic carboxylic acid or a polyhydroxy alcohol (Japanese Patent Application Laid-Open Nos. 9-24275 and 9-19637), a method of reducing a permanganate by hydrazine, a hydroxycarboxylic acid, or a salt thereof (Japanese Patent Application Laid-Open No. 6-269666), etc. Method of oxidizing manganese (II) may include an electrolytic oxidization of manganese sulfate in an aqueous solution. The oxidation of manganese (II) and the reduction of manganese (VII) may be simultaneously carried out by adding an aqueous solution of potassium permanganate into an aqueous solution of manganese sulfate (J. Chem. Soc., 2189 (1953)), etc. The manganese oxide to be used in the present invention may be prepared by any of the above methods.

The source of manganese (II) for preparing the catalyst of the present invention is selected from water-soluble salts of manganese and manganese sulfate is particularly preferable. The source of manganese (VII) is selected from water-soluble salts of manganese, and potassium permanganate and sodium permanganate are particularly preferable because these permanganates also serve as the source of alkali metal and alkaline earth metal as will be described below.

A manganese oxide used in the present invention preferably contains at least one element selected from the group consisting of alkali metals and alkaline earth metals in view of attaining high activity and selectivity and a long catalyst life. The content of alkali metals and alkaline earth metals is preferably 0.5 atom or less excluding zero, more preferably 0.05 to 0.3 atom in total per one atom of manganese. The alkali metal and alkaline earth metal may be added in the form of a water-soluble compound such as nitrate, chloride, sulfate, carbonate, phosphate and hydroxide. Preferably, these metals are added as a water-soluble alkali metal salt or alkaline earth metal salt having manganese (VII), such as potassium permanganate and sodium permanganate, because they serve as a manganese (VII) source.

The manganese oxide used in the present invention may further contain at least one metal selected from the group consisting of Ti, Zr, V, Nb, Ta, Cr, Mo, W, Zn, Ga, In, Ge, Sn and Pt. The addition of at least one of Sn, Zr and V is particularly preferable because the catalyst life is prolonged. These metals may be added in the form of nitrate, chloride, sulfate, carbonate, phosphate, hydroxide, oxide and element, and sulfate is preferable. The content of these metals is preferably 0.2 atom or less excluding zero, more preferably 0.005 to 0.1 atom in total per one atom of manganese.

The catalyst of the present invention is prepared by adding the silicon compound to the manganese oxide mentioned above. As a silicon source, any silicon compound may be used. Particularly preferred is a colloid solution of silicic anhydride, generally called as a silica sol. The mechanical strength of the catalyst increases with increasing amount of the silicon compound being added. However, an excess amount thereof tends to reduce the activity of the catalyst because the manganese oxide as the active component is diluted. Therefore, the addition amount of the silicon compound in terms of silicon dioxide is 0.1-15 % by weight, preferably 0.5-7 % by weight based on the total amount of the catalyst.

The catalyst of the present invention is prepared, for example, as described below.

As mentioned above, the manganese oxide is prepared by the oxidation of manganese (II), by the reduction of manganese (VII) or by the combination thereof. Namely, manganese (II) is oxidized in a liquid medium and/or manganese (VII) is reduced in a liquid medium to precipitate the manganese oxide. The precipitation is aged for a period of time sufficient for completing the oxidation of manganese (II) and/or the reduction of manganese (VII). Then, the precipitation is separated from the suspension containing it to obtain a wet cake of the manganese oxide. In preparing the manganese oxide, it is preferred to oxidize manganese (II) compound simultaneously with reducing manganese (VII) compound in the presence of alkali metal and/or alkaline earth metal, because the crystalline structure, the specific surface area, and the kind and amount of alkali metal and/or alkaline earth metal can be easily controlled. The wet cake of the manganese oxide thus obtained by separation is, according to known methods, washed, kneaded with the silicon compound and dried after or without shaping to obtain the catalyst of the present invention. For the separation of a wet cake of the manganese oxide from the suspension, for example, filtration and centrifugation can be used, and preferably filtration is used. Alternatively, the wet cake of the manganese oxide is immersed into a liquid containing the silicon compound thereby impregnating the manganese oxide with the silicon compound or making the silicon compound adsorbed on the manganese oxide. Then, the manganese oxide incorporated with the silicon compound is filtered off to obtain a wet cake, which is then washed and dried after or without shaping to obtain the catalyst of the present invention.

However, to improve the mechanical strength of the resultant catalyst without reducing the catalytic activity, it is preferred to add the silicon compound at any stage prior to the separation of a wet cake of the manganese oxide, thereby coprecipitating the silicon compound with the manganese oxide, impregnating the precipitated manganese oxide with the silicon compound, or adsorbing the silicon compound on the precipitated manganese oxide. In a more preferred method, the manganese (II) compound is oxidized simultaneously with reducing the manganese (VII) compounds preferably in the presence of an alkali metal and/or an alkaline earth metal, and in the presence of the silicon compound, thereby to coprecipitate the manganese oxide and the silicon compound.

In a preferred embodiment, the catalyst of the present invention may be prepared by a coprecipitation method described below.

To an aqueous solution, preferably acidic, dissolving a water-soluble manganese (II) salt and, if required, at least one water-soluble salt of a metal selected from the group consisting of Ti, Zr, V, Nb, Ta, Cr, Mo, W, Zn, Ga, In, Ge, Sn and Pt, is added an aqueous solution or a colloid solution of a silicon compound. The mixed solution thus prepared is added to an aqueous solution dissolving a manganese (VII) compound preferably having an alkali metal and/or an alkaline earth metal at 30 to 250°C under ordinary pressure or increased pressure while stirring. The manganese (II) salt and the silicon compound may be added separately from each other. For example, an aqueous solution of the manganese (II) salt is added to the aqueous solution of the manganese (VII) compound, and then, the aqueous solution or colloid solution of the silicon compound is successively added.

The precipitated manganese oxide is aged by continuing the stirring at 30 to 250°C for 1 to 10 hours to complete the oxidation of manganese (II) and the reduction of manganese (VII). Then, the precipitation is, according to known manners, filtered off, washed with water and dried after or without shaping to obtain the catalyst of the present invention in a shaped form or in the form of powder, granule and fine sphere. The shaped catlyst is used as a fixed bed catalyst and the catalyst in the form of powder, granule or fine sphere is used as a suspension bed catalyst in a batch or flow hydration of a cyanohydrin.

The cyanohydrin used for producing a hydroxycarboxylic acid amide is easily prepared by a reaction between various carbonyl compounds and hydrogen cyanide in the presence of a basic catalyst. Specifically, the cyanohydrin may include acetone cyanohydrin and lactonitrile. The hydration reaction on the catalyst of the present invention is carried out in the presence of an excess amount of water over the cyanohydrin. Namely, the content of the cyanohydrin in the starting liquid is 5-80 % by weight, preferably 20-60 % by weight. The reaction temperature is 0-120°C, preferably 10-90°C. The reaction rate is reduced when the reaction temperature is lower than the above range. A reaction temperature higher than the above range detrimentally increases the amount of by-product due to decomposition of the cyanohydrin. The hydration reaction of the present invention is preferably carried out in the presence of an oxidizing agent such as oxygen, etc. because the reduction in the catalytic activity with time can be avoided.

The present invention will be explained in more detail by reference to the following examples which should not be construed to limit the scope of the present invention.

### (A) Preparation of Catalyst

### COMPARATIVE CATALYST 1

Into a solution of 0.398 mol of potassium permanganate in 220 ml water, was quickly poured, at 70°C under stirring, a solution prepared by mixing 0.968 mol of concentrated sulfuric acid with a solution of 0.316 mol of manganese (II) sulfate monohydrate and 0.0137 mol of tin (II) sulfate in 220 ml water. Stirring was continued for 3 hours and then the resultant mixture was subjected to aging for 2 hours at 90°C. The precipitate was collected by filtration and washed with 2000 ml water in 5 portions. The cake thus obtained was extruded and dried overnight at 110°C to obtain about 60 g of a shaped catalyst having a size of 1.0 mm diameter × 3-7 mm thickness. The measured atomic ratio of the metal components of the catalyst was tin/potassium/manganese = 0.02/0.08/1.

### CATALYST 1

To 150 g wet cake prepared in the same manner as in preparing the Comparative Catalyst 1, were added 15.5 g of colloidal silica (available from Nissan Chemical Industries, Ltd. under the trade name of Snowtex 20). The resultant mixture was kneaded, extruded and dried overnight at 110°C to obtain about 60 g of a shaped catalyst having a size of 1.0 mm diameter × 3-7 mm thickness. The measured content of silicon was 5 % by weight the catalyst in terms of silicon dioxide.

### CATALYST 2

A catalyst containing 5 % by weight of silicon dioxide was prepared in the same manner as in preparing the Comparative Catalyst 1 except for pouring into the solution of potassium permanganate a liquid mixture prepared by mixing 0.968 mol concentrated sulfuric acid and 15.5 g Snowtex 20 with a solution of 0.316 mol manganese (II) sulfate monohydrate and 0.0137 mol tin (II) sulfate in 220 ml water.

### CATALYST 3

A catalyst containing 2.5 % by weight of silicon dioxide was prepared in the same manner as in preparing the Comparative Catalyst 1 except for pouring into the solution of potassium permanganate a liquid mixture prepared by mixing 0.968 mol concentrated sulfuric acid and 7.8 g Snowtex 20 with a solution of 0.316 mol manganese (II) sulfate monohydrate and 0.0137 mol tin (II) sulfate in 220 ml water.

### CATALYST 4

A catalyst containing 20 % by weight of silicon dioxide was prepared in the same manner as in preparing the Comparative Catalyst 1 except for pouring into the solution of potassium permanganate a liquid mixture prepared by mixing 0.968 mol concentrated sulfuric acid and 61 g Snowtex 20 with a solution of 0.316 mol manganese (II) sulfate monohydrate and 0.0137 mol tin (II) sulfate in 220 ml water.

### COMPARATIVE CATALYST 2

Into a solution of 0.398 mol of potassium permanganate in 220 ml water, was quickly poured, at 70°C under stirring, a solution prepared by mixing 0.968 mol of concentrated sulfuric acid with a solution of 0.316 mol of manganese sulfate monohydrate in 220 ml water. Then, the resultant mixture was subjected to aging for 2 hours at 90°C while continuing the stirring. The precipitate was collected by filtration and washed with 2000 ml water in 5 portions. The cake thus obtained was extruded and dried overnight at 110°C to obtain about 60 g of a shaped catalyst having a size of 1.0 mm diameter × 3-7 mm thickness. The measured atomic ratio of the metal components of the catalyst was potassium/manganese = 0.09/1.

### CATALYST 5

A catalyst containing 1 % by weight of silicon dioxide was prepared in the same manner as in preparing the Comparative Catalyst 2 except for pouring into the solution of potassium permanganate a liquid mixture prepared by mixing 0.968 mol concentrated sulfuric acid and 3.1 g Snowtex 20 with a solution of 0.316 mol manganese (II) sulfate monohydrate in 220 ml water.

### COMPARATIVE CATALYST 3

Into a solution of 0.398 mol of potassium permanganate in 220 ml water, was quickly poured, at 70°C under stirring, a solution prepared by mixing 0.968 mol of concentrated sulfuric acid with a solution of 0.316 mol of manganese sulfate monohydrate and 0.0137 mol of vanadyl sulfate in 220 ml water. Stirring was continued for 3 hours and then the resultant mixture was subjected to aging for 2 hours at 90°C. The precipitate was collected by filtration and washed with 2000 ml water in 5 portions. The cake thus obtained was extruded and dried overnight at 110°C to obtain about 60 g of a shaped catalyst having a size of 1.0 mm diameter × 3-7 mm thickness. The measured atomic ratio of the metal components of the catalyst was vanadium/potassium/manganese = 0.02/0.08/1 (atomic ratio).

### CATALYST 6

A catalyst containing 1.5 % by weight of silicon dioxide was prepared in the same manner as in preparing the Comparative Catalyst 3 except for pouring into the solution of potassium permanganate a liquid mixture prepared by mixing 0.968 mol concentrated sulfuric acid and 6.2 g colloidal silica (available from Nissan Chemical Industries, Ltd. under the trade name of Snowtex OUP) with a solution of 0.316 mol manganese (II) sulfate monohydrate and 0.0137 mol vanadyl sulfate in 220 ml water.

### COMPARATIVE CATALYST 4

Into a solution of 0.398 mol of potassium permanganate in 220 ml water, was quickly poured, at 70°C under stirring, a solution prepared by mixing 0.968 mol of concentrated sulfuric acid with a solution of 0.316 mol of manganese (III) sulfate monohydrate and 0.0137 mol of zirconium sulfate in 220 ml water. Stirring was continued for 3 hours and then the resultant mixture was subjected to aging for 2 hours at 90°C. The precipitate was collected by filtration and washed with 2000 ml water in 5 portions. The cake thus obtained was extruded and dried overnight at 110°C to obtain about 60 g of a shaped catalyst having a size of 1.0 mm diameter × 3-7 mm thickness. The measured atomic ratio of the metal components of the catalyst was zirconium/potassium/manganese = 0.02/0.08/1.

### CATALYST 7

A catalyst containing 4 % by weight of silicon dioxide was prepared in the same manner as in preparing the Comparative Catalyst 4 except for adding 12.2 g silica sol (available from Shokubai Kasei Kogyo, Co., Ltd. under the trade mane of CATALOID S-20L) after pouring into the solution of potassium peroxide the liquid mixture prepared by mixing 0.968 mol concentrated sulfuric acid with a solution of 0.316 mol manganese (II) sulfate monohydrate and 0.0137 mol zirconium sulfate in 220 ml water.

### (B) Measurement of Mechanical Strength

The mechanical strength of each catalyst prepared above was evaluated in terms of the degree of powdering.

In a 100 mm-diameter cylindrical drum having a circumferential surface made of a wire cloth of JIS 30 mesh, was placed 10 g of each catalyst. After rotating the drum at a speed of 150 rpm for 20 minutes, the weight of the residual catalyst in the drum was measured. The degree of powdering was determined from the equation: Degree of Powdering (% by weight) = 100 × (Sample weight (g) - Residual weight (g)) / Sample weight (g).

The results are shown in Table 1.

### (C) Measurement of Efficiency of Hydrating Acetone Cyanohydrin

The activity and selectivity of each catalyst in hydrating acetone cyanohydrin was evaluated by the conversion of acetone cyanohydrin and the selectivity of α-hydroxyisobutyramide in the following manner.

In a glass reactor tube with an inner diameter of about 9 mm equipped with a jacket, were charged 3 g of 3-4 mm broken pieces of each catalyst. Warm water of 60°C was circulated through the jacket. A starting liquid consisting of 40 parts by weight of acetone cyanohydrin, 10 parts by weight of acetone and 50 parts by weight of water was passed through the reactor tube at a flow rate of 15 g/hr The effluent liquid from the reactor tube was analyzed by a high-speed liquid chromatography 24 hours after starting the operation. The conversion of acetone cyanohydrin and the selectivity of α-hydroxyisobutyramide were determined from the results.

The conversion and selectivity thus obtained are shown in Table 1.

### (D) Measurement of Efficiency of Hydrating Lactonitrile

The activity and selectivity of each catalyst in hydrating lactonitrile was evaluated by the conversion of lactonitrile and the selectivity of lactamide in the following manner.

In a glass reactor tube with an inner diameter of about 9 mm equipped with a jacket, were charged 3 g of 3-4 mm broken pieces of each catalyst. Warm water of 40°C was circulated through the jacket. A starting liquid consisting of 35 parts by weight of lactonitrile and 65 parts by weight of water was passed through the reactor tube at a flow rate of 3 g/hr. The effluent liquid from the reactor tube was analyzed by a high-speed liquid chromatography 24 hours after starting the operation. The conversion of lactonitrile and the selectivity of lactamide were determined from the results.

The conversion and selectivity thus obtained are shown in Table 1.

**Table 1**

| | Silicon Content (wt. %) (SiO₂ basis) | Optional Metal Component | Degree of Powdering (wt. %) |
|---|---|---|---|
| Comparative Catalyst 1 | 0 | K, Sn | 3.7 |
| Catalyst 1 | 5 | K, Sn | 2.3 |
| Catalyst 2 | 5 | K, Sn | 2.3 |
| Catalyst 3 | 2.5 | K, Sn | 2.5 |
| Catalyst 4 | 20 | K, Sn | 2.0 |
| Comparative Catalyst 2 | 0 | K | 3.8 |
| Catalyst 5 | 1 | K | 2.6 |
| Comparative Catalyst 3 | 0 | K, V | 4.0 |
| Catalyst 6 | 1.5 | K, V | 2.6 |
| Comparative Catalyst 4 | 0 | K, Zr | 3.7 |
| Catalyst 7 | 4 | K, Zr | 2.3 |

**Table 1 (Contd.)**

| | Hydration Efficiency of ACH | | Hydration Efficiency of LN | |
|---|---|---|---|---|
| | Conversion of ACH (%) | Selectivity of HBD (%) | Conversion of LN (%) | Selectivity of LD (%) |
| Comparative Catalyst 1 | 80.5 | 95.5 | 99.4 | 95.0 |
| Catalyst 1 | 66.4 | 95.6 | 87.1 | 95.0 |
| Catalyst 2 | 80.0 | 95.4 | 99.3 | 95.1 |
| Catalyst 3 | 81.0 | 95.5 | - | - |
| Catalyst 4 | - | - | 87.0 | 95.0 |
| Comparative Catalyst 2 | 74.3 | 95.3 | - | - |
| Catalyst 5 | 74.4 | 95.5 | - | - |
| Comparative Catalyst 3 | 81.0 | 95.5 | - | - |
| Catalyst 6 | 81.5 | 95.6 | - | - |
| Comparative Catalyst 4 | 80.0 | 95.7 | - | - |
| Catalyst 7 | 80.0 | 95.6 | - | - |
| Note: ACH: acetone cyanohydrin LN: lactonitrile HBD: α-hydroxyisobutyramide LD: lactamide | | | | |

As described above, according to the present invention, a catalyst having a good mechanical strength and exhibiting a high activity and selectivity in hydrating a cyanohydrin is obtained. Since the catalyst of the present invention enables a stable, long-term production of a hydroxycarboxylic acid amide from the cyanohydrin, the present invention has an industrially significant advantage.

## Claims

1. A catalyst for hydrating cyanohydrin, comprising a manganese oxide as a main component and a silicon compound.

2. The catalyst for hydrating cyanohydrin according to claim 1, wherein a content of said silicon compound in terms of silicon dioxide is 0.1-15 % by weight based on said catalyst.

3. The catalyst for hydrating cyanohydrin according to claim 1, further comprising at least one element selected from the group consisting of alkali metals and alkaline earth metals.

4. The catalyst for hydrating cyanohydrin according to claim 3, wherein a content of said at least one element selected from the group consisting of alkali metals and alkaline earth metals is 0.5 atom or less, excluding zero, per one atom of manganese in said catalyst.

5. The catalyst for hydrating cyanohydrin according to claim 3, further comprising at least one element selected from the group consisting of Ti, Zr, V. Nb, Ta, Cr, Mo, W, Zn, Ga, In, Ge, Sn and Pt.

6. The catalyst for hydrating cyanohydrin according to claim 5, wherein a content of said at least one element selected from the group consisting of Ti, Zr, V, Nb, Ta, Cr, Mo, W, Zn, Ga, In, Ge, Sn and Pt is 0.2 atom or less, excluding zero, per one atom of manganese in said catalyst.

7. The catalyst for hydrating cyanohydrin according to claim 3, further comprising at least one element selected from the group consisting of Sn, Zr and V.

8. The catalyst for hydrating cyanohydrin according to claim 7, wherein a content of said at least one element selected from the group consisting of Sn, Zr and V is 0.2 atom or less, excluding zero, per one atom of manganese in said catalyst.

9. The catalyst for hydrating cyanohydrin according to any one of claims 1 to 8, wherein said catalyst is prepared by oxidizing a manganese (II) compound and/or reducing a manganese (VII) compound to form said manganese oxide, and adding said silicon compound at any stage before separating said manganese oxide from a suspension containing it.

10. The catalyst for hydrating cyanohydrin according to claim 9, wherein said manganese oxide is formed in the presence of said silicon compound.

11. The catalyst for hydrating cyanohydrin according to claim 9 or 10, wherein said manganese oxide is formed in the presence of said at least one element selected from the group consisting of alkali metals and alkaline earth metals.

12. The catalyst for hydrating cyanohydrin according to any one of claims 9 to 11, wherein said manganese oxide is formed in the presence of said at least one element selected from the group consisting of Ti, Zr, V, Nb, Ta, Cr, Mo, W, Zn, Ga, In, Ge, Sn and Pt.

13. The catalyst for hydrating cyanohydrin according to any one of claims 1 to 8, wherein said catalyst is prepared by oxidizing a manganese (II) compound simultaneously with reducing a manganese (VII) compound to form said manganese oxide, and adding said silicon compound at any stage before separating said manganese oxide from a suspension containing it.

14. The catalyst for hydrating cyanohydrin according to claim 13, wherein said manganese oxide is formed in the presence of said silicon compound.

15. The catalyst for hydrating cyanohydrin according to claim 13 or 14, wherein said manganese oxide is formed in the presence of said at least one element selected from the group consisting of alkali metals and alkaline earth metals.

16. The catalyst for hydrating cyanohydrin according to any one of claims 13 to 15, wherein said manganese oxide is formed in the presence of said at least one element selected from the group consisting of Ti, Zr, V, Nb, Ta, Cr, Mo, W, Zn, Ga, In, Ge, Sn and Pt.

17. A method of producing a hydroxycarboxylic acid amide comprising a step of hydrating a cyanohydrin in the presence of the catalyst according to any one of claims 1 to 16.
